# EUROPÄISCHE PATENTSCHRIFT

(11) **EP 1 326 551 B1**
(45) Veröffentlichungstag und Bekanntmachung des Hinweises auf die Patenterteilung: **30.08.2006**
(21) Anmeldenummer: 02767314.4
(22) Anmeldetag: 03.08.2002
(51) Int. Cl.: A61C 8/00, A61F 2/36

(54) **IMPLANTAT MIT RILLENSTRUKTUR**
IMPLANT COMPRISING A GROOVED STRUCTURE
IMPLANT PRESENTANT UNE STRUCTURE RAINUREE

(30) Priorität: 08.08.2001 DE 20113183 U; 24.07.2002 DE 20211202 U
(43) Veröffentlichungstag der Anmeldung: 16.07.2003
(73) Patentinhaber: Dinkelacker, Wolfgang, 71063 Sindelfingen (DE)
(72) Erfinder: Dinkelacker, Wolfgang, 71063 Sindelfingen (DE)
(74) Vertreter: Kindermann, Manfred
(86) Internationale Anmeldenummer: PCT/EP2002/008679
(87) Internationale Veröffentlichungsnummer: WO 2003/013383

(56) Entgegenhaltungen:
- EP-A- 0 238 860
- EP-A- 0 668 064
- EP-A- 1 013 236
- WO-A-00/06043
- FR-A- 2 573 648
- FR-A- 2 683 717
- FR-A- 2 720 268
- FR-A- 2 723 307

## Beschreibung

### Bereich der Erfindung

Die Erfindung bezieht sich auf ein operativ in den menschlichen Knochen einsetzbares Implantat mit einem Implantatkörper, der an seiner Oberfläche eine Vielzahl rillenförmiger Vertiefungen aufweist, sowie auf ein Verfahren zu seiner Herstellung.

### Stand der Technik

Die EP 1 013 236-A des Anmelders offenbart ein zylindrisches oder kegelförmiges Zahnimplantat, dessen Oberfläche eine Vielzahl in Richtung seiner Längsachse oder in einem spitzen Winkel zu dieser verlaufende rillenförmige Vertiefungen aufweist, die auch kreuzförmig angeordnet sein können. Bei einer anderen Ausführungsform dieses Implantats ist die Oberfläche des Implantatkörpers in Richtung der Längsachse in eine Anzahl Abschnitte oder Stufen unterteilt, die durch radial verlaufende Bänder voneinander getrennt sind und am Umfang eine Vielzahl der rillenförmigen Vertiefungen aufweisen. Die rillenförmigen Vertiefungen sind in ihrer Dimensionierung an die Größenordnung der Osteone des Kieferknochengewebes angepaßt, die sich an die rillenförmigen Vertiefungen anlagern. Zur Herstellung des Zahnimplantats wird ein zylindrisch, kegelförmig oder abgestufter Implantatkörper mit einer glatten Oberfläche vorgeformt, in den durch eine Materialabtragungsoperation die rillenförmigen Vertiefungen eingebracht werden. In den rillenförmigen Vertiefungen ist eine Vielzahl kleiner Kalotten dicht benachbart und räumlich verteilt angeordnet, die zur Aufnahme von Osteozyten in dem das Implantat umgebenden Kochengewebes dienen und damit den Kontakt zwischen Implantat und Knochen zusätzlich verbessern.

Sofern bei diesem Implantat der Implantatkörper kegelförmig ausgebildet ist, verlaufen die in ihrer Größe an die Osteonen des Knochengewebes angepassten Rillen innerhalb der Abschnitte parallel zur Umfangsfläche. Bedingt durch die Kegelform der Implantate verjüngen sich die Rillen über die Länge eines Abschnittes. Da die Zahl der Rillen über die Länge des Abschnitt gleich bleibt, nimmt die Tiefe und Breite der Rillen zum kleineren Umfang des Abschnitts um ein geringes Maß ab, das allein vom Kegelwinkel des Implantatkörpers abhängt.

Diese Oberflächenstruktur gestattet es den Osteonen des Knochengewebes, sich in den rillenförmigen Vertiefungen anzulagern und entlang des Implantats zu wachsen. Dadurch, dass die rillenförmigen Vertiefungen in ihrer Dimensionierung an die Größenordnung der Osteone angepaßt sind und die mit dem Implantat in Kontakt kommenden Teile der Osteone aufnehmen, wird das Einwachsen des Implantat begünstigt. Als Folge dieser Oberflächenstruktur und durch die auf das Implantat einwirkenden Drücke bildet sich im Spongiosa-Bereich des Knochens eine Compakta um das Implantat aus, die eine gute Aufnahme der auf das Implantat einwirkenden Kräfte und einen stabilen und dauerhaften Sitz des Implantats bewirkt.

Es sind des weiteren Hüftimplantate bekannt, die jeweils an der Implantatoberfläche in Längsrichtung des Implantat verlaufende Mulden oder Ausnehmungen aufweisen. FR-A-2 573 648 offenbart ein Implantat, an dessen Umfang sich eine Anzahl rillenartiger Vertiefungen über die volle Länge des Implantats erstrecken. FR-A-2 723 307 offenbart keulenförmige Mulden, die im oberen Teil der Implantatoberfläche nebeneinander angeordnet sind. FR-A-2 720 268 offenbart einzelne keulenförmige und winkelversetzte Mulden am unteren Ende des Implantats. EP-A-0 238 860 offenbart ein Hüftimplantat, das an einem Teil seines Umfangs rippenförmige Ansätze besitzt, die sich in axialer Richtung zum Teil über die volle Implantatlänge erstrecken und in ihrer Breite und Tiefe der konischen Verjüngung des Implantatschaftes folgen.

### Zusammenfassung der Erfindung

Durch die vorliegende Erfindung wird die oben in Verbindung mit EP 1 013 236-A beschriebene Wirkung weiter verstärkt. Dies wird durch eine weiter verbesserte Anpassung der Oberflächenstruktur eines Implantats der oben beschriebenen Art an den Verlauf der Osteone und deren Verhalten in der Einheilphase erreicht.

Eine erfindungsgemäße Lösung der Aufgabe, wie sie in Anspruch 1 definiert ist, sieht vor, dass die Rillen in wenigstens einem der Abschnitte im abgewickelten Umfang einen annähernd rautenförmigen Grundriß aufweisen, so dass die Rillen an beiden Rändern eines Abschnitts schmal und flach sind und dazwischen ihre maximale Breite und Tiefe aufweisen.

Gemäß einer erfindungsgemäßen Alternativlösung, wie sie in Anspruch 5 definiert ist, weisen die Rillen in wenigstens einem der Abschnitte eine sich kontinuierlich verändernde Breite und Tiefe auf, wobei der Grundriss der Rillen im abgewickelten Umfang die Form gekrümmter Segmente hat, die an beiden Rändern des besagten Abschnitts schmal und flach sind und dazwischen ihre maximale Breite und Tiefe aufweisen.

Die dadurch entstehende Oberflächenstruktur trägt der Anatomie des Knochengewebes Rechnung. Es wurde gefunden, dass die sich am Implantat anlagernden Osteone weniger in Richtung der Implantatachse verlaufen als vielmehr schräg zu dieser. Die Anlagerung der Osteone an der Implantatoberfläche erfolgt hauptsächlich unter einem spitzen Winkel, der eine räumliche Vorraussetzung für die Anlagerung vieler Osteone darstellt. Hierbei können die Osteone schräg von oben oder schräg von unten in die rillenförmigen Vertiefungen hineinwachsen. Der geneigte Verlauf der rillenförmigen Vertiefungen unterstützt diese Form der Anlagerung und verbessert Kontakt des Knochengewebes mit dem Implantat und verkürzt die Einheilphase.

Nach einem erfindungsgemäßen verfahren zur Herstellung der Rillenstruktur wird ein Rohling des Implantatkörper hergestellt, in dem durch eine Materialabtragungsoperation in wenigstens einem der Abschnitte Rillen eingebracht werden, die eine sich kontinuierlich verändernde Breite und Tiefe aufweisen, und dass die Rillen im abgewickelten Umfang einen annähernd rautenförmigen Grundriß oder die Form gekrümmter Segmente aufweisen und an beiden Rändern des besagten Abschnitts schmal und flach sind und dazwischen ihre maximale Breite und Tiefe aufweisen.

Die Materialabtragungsoperation wird vorzugsweise durch Strahlen ausgeführt bei synchronem Vorschub von Implantatkörpers und Strählwerkzeug, der von Abschnitt zu Abschnitt verändert werden kann.

Nach einem weiteren Schritt des erfindungsgemäßen verfahren wird auf die Oberfläche der Rillen eine Metallschicht durch Sputtern aufgebracht wird, die vorzugsweise eine Titanschicht sein kann.

### Beschreibung der Zeichnungen

Nachfolgend sind verschiedene Ausführungsformen der Erfindung anhand von Zeichnungen dargestellt. Es zeigen:
Figur 1 eine Ausführungsform eines Zahnimplantats gemäß der Erfindung, das einen in Abschnitte unterteilten zylindrischen Implantatkörper aufweist;
Figur 2 einen Teilschnitt entlang der Linie 2-2' in Figur 1;
Figur 3 eine schematische Darstellung einer Abwicklung eines Umfangsteils eines der Abschnitte von Figur 1;
Figur 4 einen Teilschnitt entlang der Linie 4-4' in Figur 3;
Figur 5 eine schematische Darstellung einer Abwicklung eines Umfangsteils mit Rillen, die zum Implantatkopf hin eine zunehmende Rillentiefe aufweisen;
Figur 6 eine Schnitt entlang der Linie 6-6' in Figur 5;
Figuren 7 und 8 schematische Darstellungen von Abwicklungen eines Umfangsteils des Implantats mit links oder rechts gewendelt verlaufenden Rillen;
Figuren 9 und 10 Schnitte entlang der Linien 9-9' in Figur 7 und 10-10' in Figur 8;
Figuren 11 und 12 schematische Darstellungen von Abwicklungen eines Umfangsteils des Implantats mit kreuzweise links und rechts gewendelt verlaufenden Rillen;
Figuren 13 und 14 schematische Darstellungen von Abwicklungen eines Umfangsteils des Implantats mit rautenförmig verlaufenden Rillen;
Figur 15 einen Schnitt entlang der Linie 14-14' in Figur 13;
Figuren 16 bis 18 Ausführungsformen des Implantats gemäß der Erfindung mit einem stufenförmigen Implantatkörper;
Figuren 19 und 20 Ausführungsformen des Implantats gemäß der Erfindung mit einem kegelförmigen Implantatkörper;
Figuren 21 und 22 Ausführungsformen des Implantats gemäß der Erfindung mit einem Implantatkörper, der kombinierte Rillenstrukturen aufweist;
Figuren 23 und 24 schematische Darstellungen von Abwicklungen eines Umfangsteils eines Implantats gemäß der Erfindung mit gekrümmten Rillen, die einen Grundriss in Form gekrümmter Keile aufweisen und links oder rechts gewendelt verlaufen;
Figur 25 eine schematische Darstellung der Abwicklungen eines Umfangsteils eines Implantats gemäß der Erfindung mit gekrümmten Rillen, die einen Grundriss in Form langgestreckter gekrümmter Segmente aufweisen;
Figur 26 eine schematische Darstellung der Abwicklungen eines Umfangsteils eines Implantats mit gekrümmten Rillen der in Figur 25 dargestellten Art, die kreuzförmig gewendelt verlaufen;
Figur 27 eine Ausführungsform des Implantats mit einem stufenförmigen Implantatkörper, der kombinierte Rillenstrukturen in den Figuren 25 und 26 dargestellten Art aufweist; und
Figur 28 eine Ausführungsform des Implantats gemäß der Erfindung mit einer gekrümmten Längsachse, die beispielsweise zum Ersatz von Hüftgelenken Verwendung findet.

### Detaillierte Beschreibung der in den Zeichnungen dargestellten Ausführungsbeispiele der Erfindung

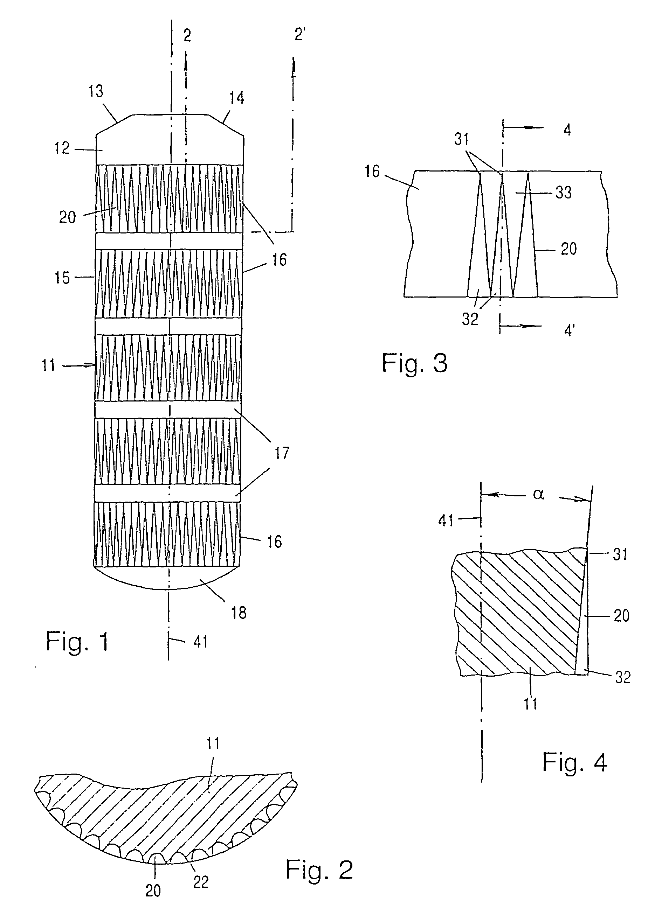

Das in Figur 1 dargestellte Zahnimplantat umfaßt einen zylindrischen Implantatkörper 11, der aus Titan, Keramik oder einem anderen mit dem Knochengewebe des menschlichen Körpers verträglichen Material ausreichender Härte besteht. Der Implantatkörper 11 weist einen Kopfbereich 12 auf, der mit Abschrägungen 13, 14 auf der Bucal- und Lingualseite ausgestattet ist, wie es in der EP-A 0 868 889 offenbart ist. Der Kopfbereich 12 dient zur Aufnahme nichtgezeigter Tragelemente für eine Zahnkrone. An den Kopfbereich 12 schließt sich ein Rumpfbereich 15 an, der in Abschnitte 16 unterteilt ist. Zwischen des Abschnitten befinden sich Bänder 17. Das in Figur 1 dargestellte Beispiel weist fünf Abschnitte 16 auf, die durch vier Bänder 17 getrennt sind. Nach unten wird der Implantatkörper 11 durch einen abgerundeten Fußbereich 18 abgeschlossen.

Die Abschnitte 16 weisen an ihrem Umfang rillenförmige Vertiefungen 20 auf, hierin kurz Rillen genannt, die dicht nebeneinander angeordnet sind und in Bezug auf den Umfang des Implantatkörpers 11 parallel zur Implantatachse verlaufen. Die Rillen 20 haben vorzugsweise ein konkaves Profil, das an seinen Rändern in den Umfang des Rumpfbereiches 15 ausläuft und am Ort der größten Tiefe mit jedem der beiden benachbarten Vertiefungen einen abgerundeten Kamm 22 bildet (Schnittdarstellung von Figur 2). Die Rillen 20 haben eine Breite, die vorzugsweise im Bereich zwischen 0 und 300 Mikrometern liegt, und eine variable Tiefe zwischen 0 und vorzugsweise bis zu 150 Mikrometern.

Die Figur 3 zeigt eine schematische Darstellung einer Abwicklung eines der Abschnitte 16, in der drei Rillen 20 dargestellt sind. Die Rillen 20 bilden einen Winkel α radial zur Längsachse 41 des Implantatkörpers 11 und weisen über ihre Länge eine unterschiedliche Tiefe auf (Figur 4). Hierdurch entsteht in Abwicklungsebene ein annähernd keilförmiger Grundriß der Rillen 20. Am oberen Rand des dargestellten Abschnitts 16 liegt die Spitze 31 der Begrenzungslinien auf dem Durchmesser des Umfangs. Am unteren Rand des Abschnitts im Bereich 32 hat die Keilform ihre größte Breite und zugleich hat dort die Rille ihre größte Tiefe, wie der Schnitt von Figur 4 zeigt. Zwischen den Rillen 20 befinden sich in umgekehrter Richtung annähernd keilförmig verlaufende Stege 33, die sich zu den Spitzen 31 der Rillen 20 hin verbreitern und deren Höhe den Umfang der Abschnitte bestimmen. Es versteht sich von selbst, das die Begrenzungslinien der Rillen 20 in der realen Abwicklung des Umfangs in die Abbildungsebene Krümmungen zeigen, die in den Figuren 3 und 4 aus Gründen der einfacheren Darstellung nicht gezeigt sind.

Der Winkel α wird durch die maximale Tiefe der Rillen und durch die axiale Länge der Abschnitte 16 bestimmt. Bei einer maximalen Rillentiefe vom 150 Mikrometern und einer Abschnittslänge von ca. 2 mm ergibt sich für α ein Wert von annähernd 4,5 Grad.

Die beschriebene Oberflächenstruktur des Implantats trägt der Anatomie des Kieferknochengewebes Rechnung. Während der Einheilphase des Implantats können sich die Osteone des das Implantat umgebenden Knochengewebes in den Rillen 20 anlagern. Es wurde gefunden, dass die sich am Implantat anlagernden Osteone weniger in Richtung der Implantatachse verlaufen als vielmehr schräg zu dieser. Die Anlagerung der Osteone an der Implantatoberfläche erfolgt hauptsächlich unter einem spitzen Winkel, der eine räumliche Voraussetzung für die Anlagerung vieler Osteone darstellt. Hierbei können die Osteone schräg von oben oder schräg von unten in die Rillen 20 hineinwachsen. Der geneigte Verlauf der Rillen 20 begünstigt diese Form der Anlagerung und verbessert den innigen Kontakt des Kieferknochengewebes mit dem Implantat und verkürzt die Einwachszeit.

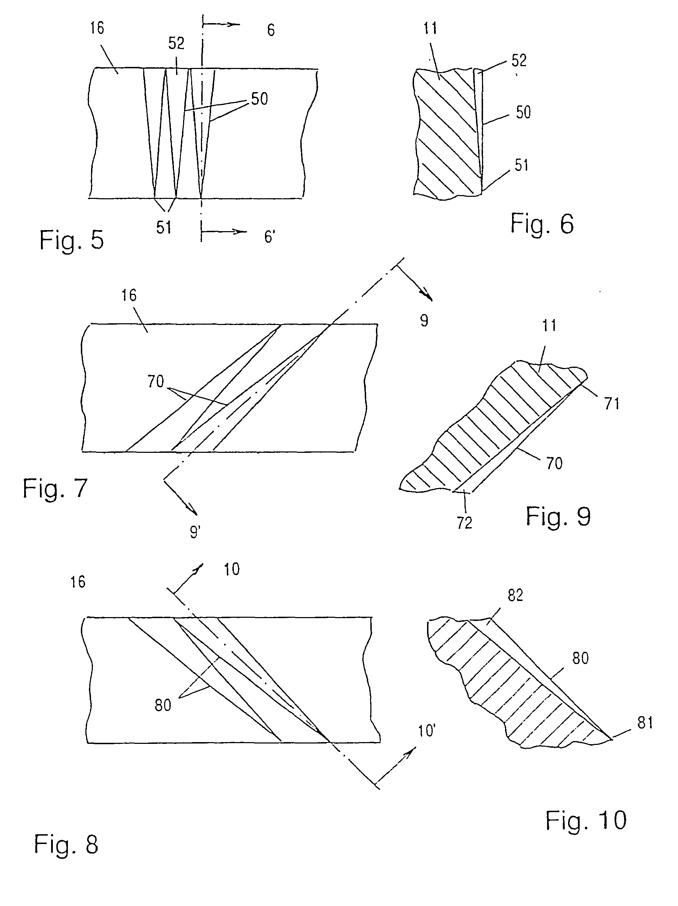

Abweichend von der in den Figuren 1 bis 4 dargestellten Form können die Rillen anders angeordnet sein oder eine andere Form aufweisen. Die Figuren 5 und 6 zeigen einen Rillenverlauf von Rillen 50, der gegenüber dem in den Figuren 3 und 4 dargestellten Rillenverlauf um 180 Grad verdreht ist, d.h. dass die Spitze 51 der Rillen 50 am unteren Rand des dargestellten Abschnitts 16 liegt und der Bereich 52 der größten Rillentiefe am oberen Rand dieses Abschnitts 16 liegt, wie die Schnittdarstellung von Figur 6 zeigt.

Die Figuren 7 und 8 zeigen die Abwicklungen von Abschnitten 16 des Implantatkörpers 11, in denen die Rillen in Bezug auf den Umfang des Implantatkörpers 11 wendelförmig verlaufen.
Die Figur 7 zeigt einen rechtsgewendelten Verlauf von Rillen 70, die zudem keilförmig vom oberen Rand 71 des Abschnitts 16 zum unteren Rand dieses Abschnitts verlaufen, wo der Bereich 72 der größten Tiefe der Rillen 70 liegt (Figur 9). Die Figur 8 zeigt einen linksgewendelten Verlauf von Rillen 80, die vom unteren Rand 81 des Abschnitts 16 zum oberen Rand des Abschnitts verlaufen, wo in diesem Falle der Bereich 82 der größten Tiefe der Rillen 80 liegt, wie in der Schnittdarstellung von Figur 10 gezeigt.

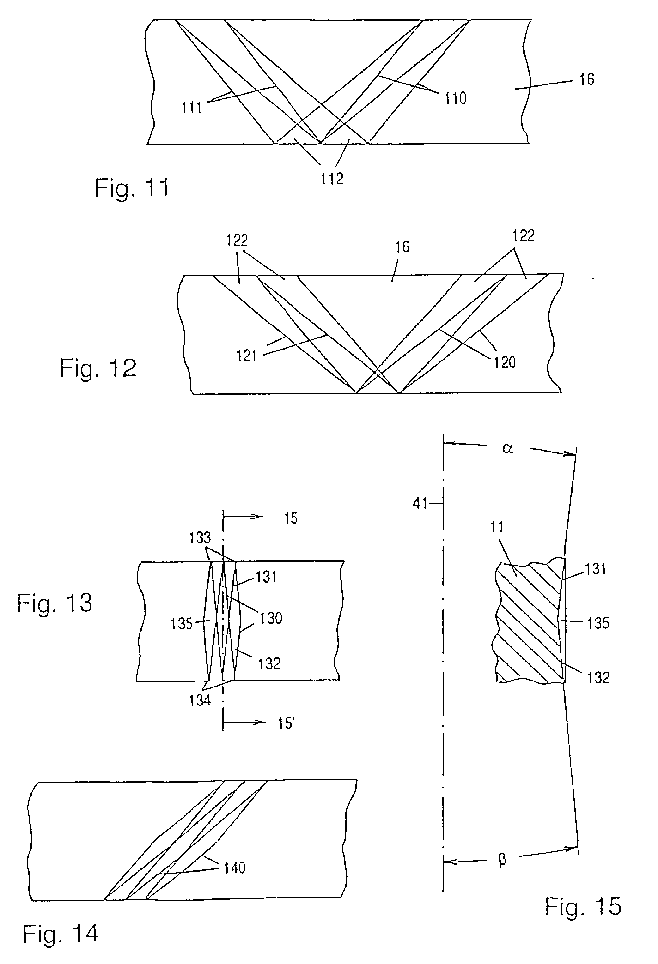

In den Figuren 11 und 12 sind Abwicklungen von einem kreuzweisen Verlauf der Rillen dargestellt. Bei der Ausführung gemäß Figur 11 sind erste Rillen 110 vorgesehen, die in einer ersten Richtung mit einem spitzen Steigungswinkel linksgewendelt verlaufen, und zweite Rillen 111 vorgesehen, die in einer zweiten Richtung mit einem spitzen Steigungswinkel rechtsgewendelt verlaufen und sich mit den ersten Rillen 110 kreuzen. Die Rillen 110 und 111 haben einen keilförmigen Verlauf vom oberen Rand des Abschnitts 112 zum unteren Rand dieses Abschnitts, wo der Bereich 112 der größten Tiefe der Rillen 110 und 111 liegt. Durch diesen Rillenverlauf werden die in Figur 3 dargestellten Stege 33 geschnitten, so dass eine Vielzahl noppenförmiger Erhöhungen entsteht, deren Höhe sich bis zum Umfang der Abschnitte 16 erstreckt bzw. diesen bestimmt.

In der Ausführung gemäß Figur 12 haben Rillen 120 und 121 einen kreuzweisen verlauf wie die Rillen 110 und 111 in Figur 11, jedoch sind die Rillen 120 und 121 so angeordnet, dass der keilförmige Verlauf vom unteren Rand des Abschnitts 122 zum oberen Rand dieses Abschnitts erfolgt, wo der Bereich der größten Tiefe der Rillen 120 und 121 liegt.

In der Ausführungsform nach den Figuren 13 bis 15 weisen die Rillen in der Abwicklung des Umfangs einen annähernd rautenförmigen Grundriß auf, der sich aus zwei gegeneinander gerichteten keilförmigen Rillenpaaren ergibt. Die Figur 13 zeigt die Abwicklung eines Abschnitts 16 des Implantatkörpers 11 mit Rillen 130, deren Begrenzungslinien annähernd rautenförmig verlaufen. Die Rillen 130 sind dicht nebeneinander angeordnet und in Bezug auf den Umfang des Implantatkörpers parallel zur Implantatachse ausgerichtet. Jede der Rillen 130 setzt sich aus zwei keilförmigen Rillenteilen 131, 132 zusammen, von denen der Rillengrund der einen Rillenteils 131 einen Winkel α mit der Längsachse 41 Implantats bildet und der Rillengrund der anderen Rillenteils 132 einen Winkel β mit der Längsachse 41 des Implantats bildet (Figur 15). Die beiden äußeren Spitzen 133 und 134 einer jeden der Rillen 130 liegen somit bei dieser Ausführungsform auf der Umfangslinie des Abschnitts 16, und der Bereich 135 der maximalen Tiefe einer jeden der Rillen 130 liegt in ihrer Mitte, wo die beiden Rillenteile 131 und 132 zusammentreffen.

Die Figur 14 zeigt die Abwicklung eines Abschnitts 141 des Implantatkörpers mit Rillen 140, deren Begrenzungslinien ebenfalls rautenförmig ausgebildet sind. Die Rillen 140 verlaufen jedoch in Bezug auf den Umfang des Implantatkörpers wendelförmig, wie es oben mit Bezug auf die Figuren 8 und 9 beschrieben wurde. Im übrigen entsprechen die Rillen 140 in ihrer Form den Rillen 130 von Figur 13.

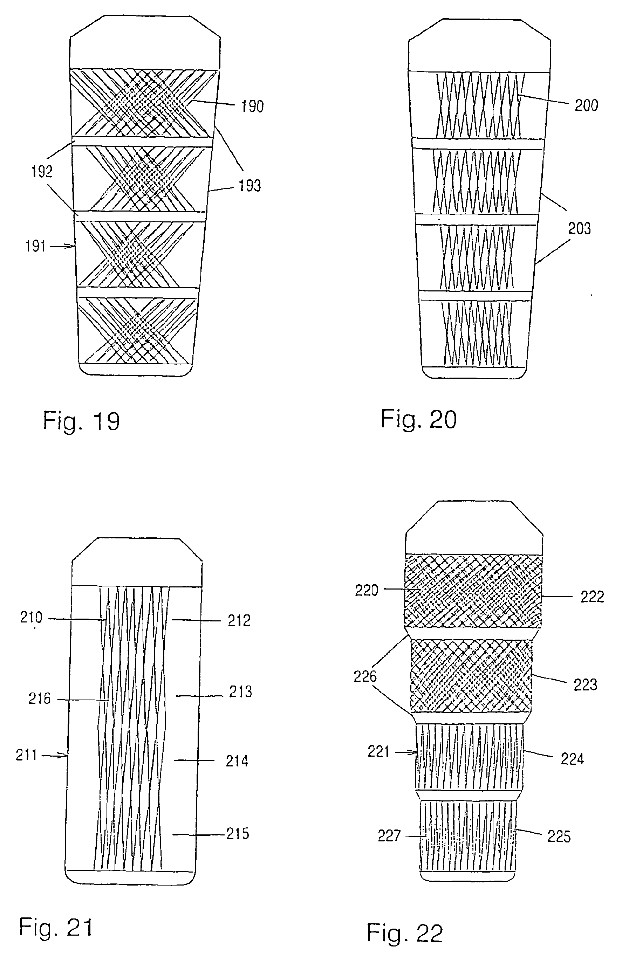

Die in den Figuren 5 bis 15 gezeigten Rillenstrukturen bieten günstige vorraussetzungen für die Anlagerung der Osteone während der Einheilphase und bewirken eine zusätzliche Sicherung gegen axiale Verlagerung und Verdrehung des Implantats im eingewachsenen Zustand. Die verschiedenen Rillenstrukturen können in vorteilhafter Weise je nach Bedarf in einem Implantat kombiniert werden, wie z.B. anhand der Figuren 21 und 22 beschrieben wird.

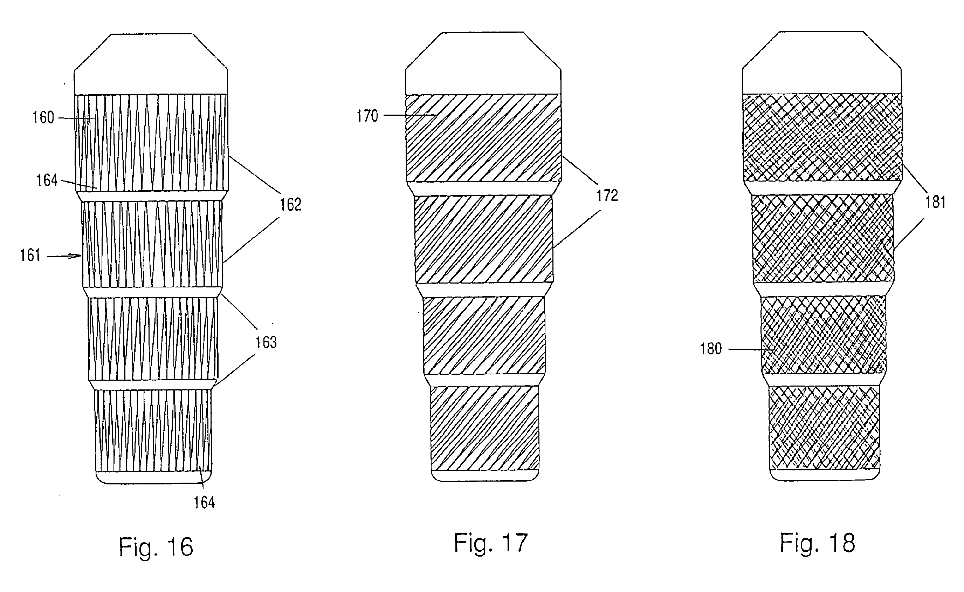

Nachfolgend werden anhand der Figuren 16-22 verschiedene Ausführungsformen von Zahnimplantaten dargestellt, bei denen Rillenstrukturen der oben beschriebenen Art Anwendung finden. Die dargestellten Zahnimplantate werden vorzugsweise aus Titan hergestellt.

Die Figuren 16 bis 18 zeigen Ausführungsformen von Zahnimplantaten, bei denen der Implantatkörper eine Abstufung des Durchmessers aufweist. Es kann eine Vielzahl von Durchmesserstufen vorgesehen sein, wobei die am Durchmesser gemessene Stufenhöhe vorzugsweise in der Größenordnung von 20 bis 300 Mikrometern liegen kann. Die in Figur 16 dargestellte Ausführungsform weist einen Implantatkörper 161 mit vier Stufen 162 auf. Zwischen den Stufen befinden sich konisch verlaufende Übergangsbereiche 163, hierin auch Bänder genannt, die im Verhältnis zur Länge der Stufen schmal sind. Der Umfang einer jeden Stufe 162 ist mit einer Rillenstruktur nach Art der in den Figuren 3 bis 15 dargestellten Rillen 20 versehen. Am Umfang einer jeden Stufe 162 sind in Bezug auf den Umfang in Richtung der Längsachse des Implantatkörpers 11 verlaufende Rillen 160 vorgesehen nach Art der in Verbindung mit den Figuren 3 und 4 beschriebenen Rillen 20. Die Rillen 160 haben im Bereich 164 größter Tiefe alle die gleiche Breite, so dass sich eine unterschiedliche Anzahl Rillen 160 in jeder Stufe 162 ergibt. Beim Implantat nach Figur 17 sind am Umfang von Stufen 172 rechtsgewendelte Rillen 170 angeordnet nach Art der in Verbindung mit den Figuren 7 und 9 beschriebenen Rillen 70. Das Implantat nach Figur 17 entspricht im übrigen dem Implantat von Figur 16. Das Implantat gemäß Figur 18 unterscheidet sich vom Implantat gemäß Figur 17 dadurch, dass der Umfangsbereich einer jeden Stufe 181 mit kreuzförmig verlaufenden Rillen 180 nach Art der in Verbindung mit den Figuren 11 und 12 beschriebenen Rillen 111 ausgestattet ist. In den Figuren 16 bis 18 sind die Rillen 160, 170 und 180 lediglich schematisch dargestellt.

Die Figuren 19 und 20 zeigen Ausführungsformen von Zahnimplantaten, die einen kegelförmigen Implantatkörper aufweisen. Bei der Ausführung von Figur 19 ist der kegelförmige Implantatkörper 191 durch entlang Umfangs verlaufende Bänder 192 in Abschnitte 193 unterteilt. Am Umfang der Abschnitte 193 befinden sich kreuzförmig verlaufende Rillen 190 nach Art der in Verbindung mit den Figuren 11 und 12 beschriebenen Rillen 111. Die Figur 20 zeigt eine Ausführung, bei die Abschnitte rautenförmige verlaufende Rillen 200 aufweisen nach Art der in Verbindung mit den Figuren 13 bis 15 beschriebenen Rillen. Das Implantat nach Figur 20 entspricht im übrigen dem Implantat von Figur 19. In den Figuren 19 und 20 sind die Rillen 190 und 200 lediglich teilweise dargestellt; auch bei diesen Ausführungsbeispielen erstreckt sich die beschriebene Rillenstruktur jeweils über den ganzen Umfang der Abschnitte 193 und 203.

Die Figuren 21 und 22 zeigen Zahnimplantate, bei denen jeweils ein Teil der Abschnitte unterschiedliche Rillenstrukturen aufweisen. Die Figur 21 zeigt ein Implantat mit einem zylindrischen Implantatkörper 211, an dessen Umfang Reihen von Rillen 210 angeordnet sind, durch die der Implantatkörper in vier Abschnitte 212, 213, 214 und 215 unterteilt ist. Die Rillen des obersten Abschnitts 212, der dem Implantatkopf am nächsten liegt, weist an seinem Umfang keilförmige Rillen 210 nach Art der in Verbindung mit den Figuren 3 und 4 beschriebenen Rillen 20 auf. Der in Richtung des Implantatfußes folgende Abschnitt 213 weist an seinem Umfang Rillen 216 mit einer umgekehrten Keilform auf nach Art der in Verbindung mit den Figuren 5 und 6 beschriebenen Rillen 50. Hieran schließt sich der Abschnitt 214 an, der wiederum die gleiche Rillenform aufweist wie der Abschnitt 212, und danach folgt der Abschnitt 215 mit Rillen der umgekehrten Keilform. Die Abschnitte 212 bis 216 gehen fugenlos ineinander über und werden nur durch den Wechsel der Rillenstruktur markiert. Auch in Figur 21 ist aus Darstellungsgründen nur ein Teil der Rillen gezeigt.

In Figur 22 ist eine Ausführungsform eines Zahnimplantats nach Art der in Verbindung mit den Figur 16-18 beschriebenen stufenförmigen Implantate dargestellt. Bei dieser Ausführungsform weist der Implantatkörper 221 Gruppen von Abschnitten auf, wobei die Rillenstruktur der einen Gruppe von der der anderen Gruppe abweicht. Der Implantatkörper 221 ist in vier Stufen 222-225 unterteilt, die durch konische Übergangsbereiche 226 miteinander verbunden sind. Die beiden obersten Stufen 222 und 223 sind an ihrem Umfang mit kreuzweise verlaufenden Rillen 210 nach Art der anhand der Figuren 11 oder 12 beschriebenen Rillen ausgestattet. Die in Richtung des Implantatfußes folgenden Abschnitte 224-225 weisen an ihrem Umfang Rillen 227 auf, die in Bezug auf den Umfang des Implantatkörper 221 eine in Längsrichtung des Implantats verlaufende Keilform nach Art der in Verbindung mit den Figuren 3 und 4 beschriebenen Rillen 20 haben.

Durch Verwendung einer die Stufen wechselnder Oberflächenstrukturen auf dem gleichen Implantat kann einerseits die unterschiedliche Dichte des Knochens berücksichtigt werden, der das Implantat über dessen Länge umgibt. So ermöglichen z.B. beim Implantat nach Figur 22 die beiden obersten Stufen 222 und 223 mit ihren kreuzweise verlaufenden Rillen eine gute Verankerung des Implantats innerhalb der Compakta und dem angrenzenden Bereich, während die Oberflächen der folgenden Stufen dem nach unten zunehmend poröseren Knochengewebe angepaßt werden können. Die Kombination unterschiedlicher Oberflächenstrukturen über die Länge des Implantatkörpers unterstützt auch das Ziel, das Implantat während der Einheilphase und im eingeheilten Zustand gegen axiale Verlagerungen und Verdrehungen zu sichern und den Druck besser in den Knochen einzuleiten.

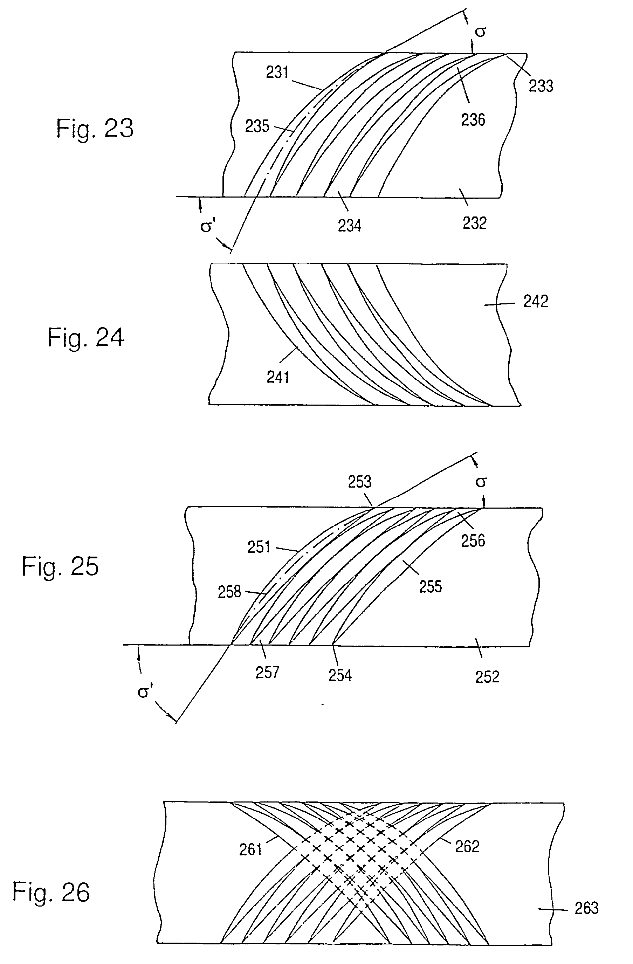

Beispiele weiterer Ausbildungen der Erfindung sind in den Figuren 23 bis 28 dargestellt. Diese Ausführungen weisen gekrümmte Rillen in Keilform oder in Form langgezogener Segmente auf, die gewendelt oder kreuzweise gewendelt verlaufen.

Die Figur 23 zeigt die Abwicklung des Umfangs eines Implantatabschnitts 232 mit rechtsgewendelten Rillen 231, die in der Abwicklung einen Grundriss aufweisen, der die Form eines gekrümmten oder gebogenen Keils besitzt. Der Grad der Krümmung dieses Keils geht über die oben erwähnten Krümmungen der Begrenzungslinien der geradlinigen Rillen 20 hinaus, die in der realen Abwicklung des Umfangs in die Abbildungsebene vorhanden sind und die in den Figuren 3 bis 14 aus Gründen der einfacheren Darstellung nicht gezeigt sind. Der Tiefenverlauf der Rillen 231 entspricht dem in Figur 4 und 9 dargestellten Tiefenverlauf der Rillen 20 und 70. Am oberen Rand des dargestellten Abschnitts 232 liegt die Spitze 233 des gekrümmten Keils auf dem Durchmesser des Umfangs. Am unteren Rand des Abschnitts 232 liegt der Bereich 234, wo der gekrümmte Keil seine größte Breite und zugleich seine größte Tiefe aufweist. Zwischen den dicht benachbarten und aneinander angrenzenden Rillen 231 befinden sich in umgekehrter Richtung entsprechend gekrümmt verlaufende keilförmige Stege 236, die sich zu den Spitzen 233 der Rillen 231 hin verbreitern und deren Höhe den Umfang des Abschnitts 232 bestimmt. Die Längsachsen 235 der Rillen 231 bilden im Bereich 233 der geringsten Rillentiefe einen Winkel σ mit der oberen Begrenzung des Abschnitts 232, und ihre Längsachsen 235 bilden im Bereich 234 der größten Rillentiefe einen Winkel σ' mit der unteren Begrenzung des Abschnitts 232, wobei σ < σ' ist.

Die Figur 24 zeigt die Abwicklung des Umfangs eines Implantatabschnitts 242 mit linksgewendelten Rillen 241, die wie die Rillen 231 einen Grundriss aufweisen, der die Form eines gekrümmten oder gebogenen Keils besitzt. Im übrigen entsprechen die Rillen 241 in Form und Anordnung den Rillen Rillen 231 von Figur 23.

Die Figur 25 zeigt die Abwicklung eines Abschnitts 252 eines Implantatkörpers mit Rillen 251, deren Grundriss die Form langgestreckter bananenähnlich gekrümmter Segmente aufweist. Die Rillen 251 sind dicht nebeneinander angeordnet und verlaufen in Bezug auf den Umfang des Implantatkörpers wendelförmig, wie oben z.B. mit Bezug auf Figur 14 beschrieben wurde. Jede der Rillen 251 kann aus zwei fließend ineinander übergehenden Rillen 231, 241 bestehend angesehen werden. Für den Tiefenverlauf gilt das für diese Rillen Ausgeführte. Die beiden äußeren Spitzen 253 und 254 einer jeden der Rillen 251 liegen auf der Umfangslinie des Abschnitts 252, und der Bereich maximaler Tiefe einer jeden der Rillen 251 kann annähernd in der Mitte ihrer Längsausdehnung liegen, wie in Figur 25 für den Bereich 255 dargestellt. Der Bereich maximaler Tiefe einer jeden der Rillen 251 kann auch außerhalb der Rillenmitte wie z.B. im ersten oder letzten Viertel oder im ersten oder letzten Drittel der Längsausdehnung der Rillen 251 liegen.

zwischen benachbarter Rillen 251 liegen entsprechend keilförmig gekrümmte Stege 256 und 257, die sich zu den Spitzen 253, 254 der Rillen 251 hin verbreitern und deren Höhe den Umfang des Abschnitts 252 bestimmt. Die Längsachsen 258 der Rillen 251 bilden im Bereich 253 einen Winkel σ mit der oberen Begrenzung des Abschnitts 252 und im Bereich 254 einen Winkel σ' mit der unteren Begrenzung des Abschnitts 252, wobei σ < σ' ist.

Die Figur 26 zeigt die Abwicklung eines Abschnitts 263 eines Implantatkörpers mit Rillen 261, 262, deren Grundriss die Form langgestreckter bananenähnlich gekrümmter Segmente nach Art der Rillen 251 von Figur 25 aufweist. Die Rillen 261, 262 verlaufen kreuzweise zueinander, wie es für die Rillen 111 und 121 in Verbindung mit den Figuren 11 und 12 beschrieben wurde. Bei der Ausführung gemäß Figur 26 verlaufen die Rillen 261 linksgewendelt und kreuzen sich mit den Rillen 262, die ihrerseits rechtsgewendelt verlaufen. Der Tiefenverlauf der Rillen 261 und 262 entspricht dem Tiefenverlauf der gekrümmten Rillen 251 wie er in Verbindung mit Figur 25 beschrieben wurde. Durch diesen Rillenverlauf werden die zwischen benachbarten Rillen 261, 262 befindlichen Stege nach Art der Stege 256 und 257 geschnitten, so dass eine Vielzahl noppenförmiger Erhöhungen entsteht, deren Höhe sich bis zum Umfang des Abschnitts 263 erstreckt bzw. diesen bestimmt.

Die in den Figuren 23 bis 26 dargestellten Rillenstrukturen haben den zusätzlichen Vorteil, dass die horizontal verlaufenden Osteone der Compakta und die vertikalen Osteone der Spongiosa ineinander übergehen.

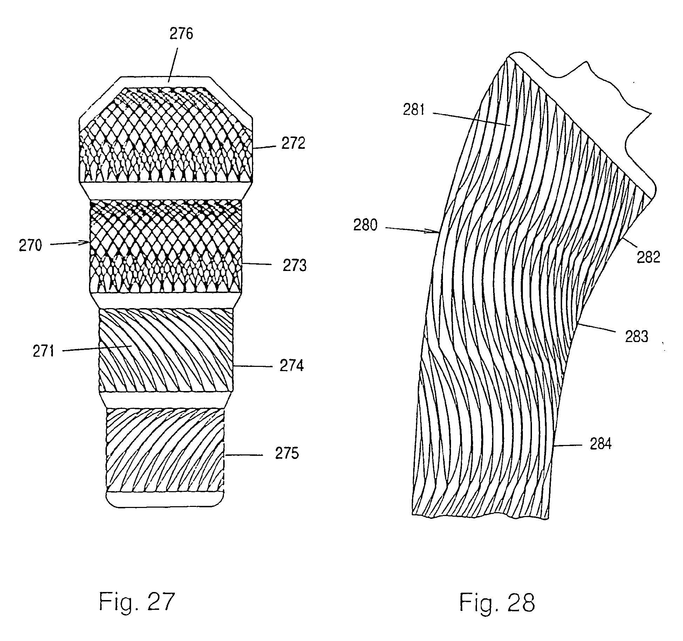

Die Rillenstrukturen gemäß Figuren 23 bis 26 können je nach Bedarf in einem Implantat kombiniert werden. Die Figur 27 zeigt hierfür ein Beispiel anhand eines Zahnimplantats 270 nach Art der oben in Verbindung mit den Figuren 16 bis 18 und 22 beschriebenen Implantatausführungen, bei denen der Implantatkörper als gestufter Kegel ausgebildet ist. Das Implantat 270 umfaßt vier Abschnitte 272 bis 275, von denen die oberen Abschnitte 272, 273 kreuzweise gewendelte Rillen nach Art der Rillen 261 und 262 von Figur 26 aufweisen. Hierbei erstrecken sich sich die Rillen 271 des obersten Abschnitts 272 in den Kopfbereich des Implantats 270 bis zu einem schmalen Randbereich 276, der annähernd parallel zum oberen Rand des Implantats verläuft. Die Abschnitte 274, 275 besitzen links- und rechtsgewendelte Rillen 271 der Art wie in Figur 25 dargestellt.

Des weiteren können die Rillenstrukturen gemäß Figuren 23 bis 26 bei zylindrischen Zahnimplantaten nach Art der in den Figuren 1 und 21 dargestellten Implantate verwendet werden sowie bei kegelförmigen Zahnimplantaten nach Art der in den Figuren 19 und 20 dargestellten Implantate

Die Erfindung kann bei Implantaten Anwendung finden, deren Implantatkörper nicht rotationssymmetrisch ist oder eine gekrümmte Längsachse aufweist, wie dies z.B. bei Hüftgelenk-Implantaten der Fall sein kann. Die Figur 28 zeigt einen Teil 281 eines solchen Implantats, das eine gekrümmt verlaufende Längsachse aufweist, entlang der Abschnitte 282, 283 und 284 angeordnet sind, von denen jeder einen keilförmigen Querschnitt aufweist und die fugenlos aneinander anschließen. Die Abschnitte 282-284 besitzen Rillen 281 nach Art der Rillen 251 in Figur 25, die im kopfseitigen Abschnitt 282 linksgewendelt, im darauffolgenden Abschnitt 283 rechtsgewendelt und im nächsten Abschnitt 284 wiederum linksgewendelt verlaufen. Der dargestellte Teil 281 kann im Bereich der Abschnitte 282-284 einen unrunden Querschnitt aufweisen, dem die Rillen in Länge, Neigung und Krümmung entsprechend angepasst sind.

Die Oberfläche des erfindungsgemäßen Implantats weist eine durch Sputtern hergestellte Metallschicht auf, durch die die Oberfläche eine das Anlegen der Osteonen fördernde Rauhigkeit erhält. Die Metallschicht besteht vorzugsweise aus Titan. Die Titanschicht überdeckt einerseits die Oberfläche der Rillen der in Verbindung mit den Figuren 2 bis 15 oder 23 bis 26 beschriebenen Ausführungsformen und andererseits die Zwischenräume zwischen den Rillen, wozu auch die Oberflächen der Stege 33, 236, 256 und 257 bei Implantaten der in Verbindung mit den Figuren 3, 23 und 25 beschriebenen Art gehören, sowie die zwischen den Abschnitten befindlichen radial verlaufenden Bänder, wie sie bei den in Figuren 3, 16-20, 22 und 27 dargestellten Ausführungsformen Verwendung finden. Eine so behandelte Oberfläche hat den zusätzlichen Vorteil, dass sich die Osteoziten der Osteone sowohl im Bereich der Rillen als auch außerhalb der Rillen in die durch den Sputterprozess hergestellten Mulden der Metallschicht einlagern können.

Ein bevorzugtes Verfahren zur Herstellung von Implantaten gemäß der Erfindung besteht darin, dass in bekannter Weise ein Rohling des Implantatkörpers als Zylinder, Kegel oder abgestufter Kegel oder also ein Implantatkörper mit gekrümmter Längsachse jeweils mit einer glatten Oberfläche hergestellt wird. Danach wird auf den Implantatkörper eine Materialabtragungsoperation angewendet, durch die in wenigstens einem Teil der Abschnitte wahlweise Rillen der in den Figuren 2 bis 15 oder 23 bis 26 dargestellen Art erzeugt werden. Die Materialabtragungsoperation besteht vorzugsweise aus einem Strählvorgang, in dem Verlauf, Form und Abmessung der Rillen durch synchronen Vorschub von Implantatkörper und Strählwerkzeug erzeugt wird. So kann der synchrone Vorschub so gewählt werden, dass keilförmige Rillen hergestellt werden, die über einen Teil eines Abschnitts radial zur Längsachse des Implantatkörpers einen ersten Winkel α bilden und über einen weiteren Teil des gleichen Abschnitts radial zur Längsachse des Implantatkörpers einen zweiten Winkel β bilden. Des weiteren kann der synchrone Vorschub so gewählt werden, dass die hergestellten Rillen rautenförmig oder gekrümmt verlaufen oder die Form von langgestreckten bananenähnlichen Segmenten haben und in Bezug auf den Umfang des Implantats axial, gewendelt oder kreuzweise gewendelt verlaufen. Bei der Herstellung der Rillen in aufeinanderfolgenden Abschnitten des Implantats kann der synchrone Vorschub von Abschnitt zu Abschnitt verändert werden, um auf dem gleichen Implantat eine Kombination von Abschnitten mit unterschiedlicher Rillenstruktur zu erzeugen. Hierbei kann die Rillenstruktur auf den Kopfbereich des Implantats 270 ausgedehnt werden bis zu einem schmalen Randbereich 276, der annähernd parallel zum oberen Rand des Implantats verläuft.

In einem weiteren Schritt wird auf die Oberfläche des erfindungsgemäßen Implantats eine Metallschicht aufgebracht wird, durch die die Oberfläche eine das Anlegen der Osteonen fördernde Rauhigkeit erhält. Dies geschieht durch Sputtern von Metall auf die Oberfläche des Implantats. Als Sputtermetal wird vorzugsweise Titan verwendet. Die Titanschicht überdeckt die Oberfläche der Rillen des erfindungsgemäßen Implantats sowie die Zwischenräume zwischen den Rillen einschließlich der Stege 33, 236, 256 und 257 bei Implantaten der in Verbindung mit den Figuren 3, 23 und 25 beschriebenen Art, die zwischen den Abschnitten befindlichen radial verlaufenden Bänder bei Implantaten der in Verbindung mit den Figuren 3, 16-20, 22 und 27 beschriebenen Art und den Kopfbereich 13, 276.

Während die Erfindung anhand bevorzugter Ausführungsformen dargestellt und beschrieben wurde, können weitere Abwandlungen und andere Ausführungsformen der Erfindung realisiert werden, ohne dass dadurch der durch die Ansprüche definierte Bereich der Erfindung verlassen wird.

## Patentansprüche

1. Knochenimplantat, insbesondere Zahnimplantat, mit einem Implantatkörper (11), der an seinem Umfang eine Vielzahl in Richtung der Längsachse des Implantatkörpers oder in einem spitzen Winkel zu dieser verlaufende Rillen (20) aufweist, deren Größenordnung der Größenordnung der Osteone des Knochengewebes entspricht, die sich an die rillenförmigen Vertiefungen anlagern können, und dessen Oberfläche in Richtung seiner Längsachse in eine Anzahl Abschnitte (16) unterteilt ist, **dadurch gekennzeichnet, dass** die Rillen (130, 140, 200) in wenigstens einem der Abschnitte im abgewickelten Umfang einen annähernd rautenförmigen Grundriß aufweisen, so dass die Rillen (251) an beiden Rändern eines Abschnitts schmal und flach sind und dazwischen ihre maximale Breite und Tiefe aufweisen.

2. Implantat nach Anspruch 1, **dadurch gekennzeichnet, dass** zwischen den Abschnitten (203) in Umfangsrichtung verlaufende Bänder (192) angeordnet sind, in deren Umfang die flachen Enden der Rillen (200) auslaufen.

3. Implantat nach Anspruch 1, **dadurch gekennzeichnet, dass** die Rillen benachbarter Abschnitte (212-215) aneinander anschließen.

4. Implantat nach Anspruch 1, **dadurch gekennzeichnet, dass** der Grund der Rillen (130) über einen Teil eines Abschnitts (16) in einen ersten Winkel α radial zur Längsachse (41) des Implantatkörpers (11) geneigt ist und über einen weiteren Teil des gleichen Abschnitts (16) in einem zweiten Winkel β radial zur Längsachse (41) geneigt ist, der dem ersten Winkel entgegengesetzt gerichtet ist.

5. Knochenimplantat, insbesondere Zahnimplantat, mit einem Implantatkörper (11), der an seinem Umfang eine Vielzahl in Richtung der Längsachse des Implantatkörpers oder in einem spitzen Winkel zu dieser verlaufende Rillen (20) aufweist, deren Größenordnung der Größenordnung der Osteone des Knochengewebes entspricht, die sich an die rillenförmigen Vertiefungen anlagern können, und dessen Oberfläche in Richtung seiner Längsachse in eine Anzahl Abschnitte (16) unterteilt ist, **dadurch gekennzeichnet, dass** die Rillen (251, 261, 262) in wenigstens einem der Abschnitte (252, 242) eine sich kontinuierlich verändernde Breite und Tiefe aufweisen, und dass der Grundriss der Rillen im abgewickelten Umfang die Form gekrümmter Segmente aufweist, die an beiden Rändern des besagten Abschnitts schmal und flach sind und dazwischen ihre maximale Breite und Tiefe aufweisen.

6. Implantat nach Anspruch 1 oder 5, **dadurch gekennzeichnet, dass** die Rillen (251) ihre maximale Breite und Tiefe in einem Bereich (255) aufweisen, der annähernd in der Mitte ihrer Längsausdehnung liegt.

7. Implantat nach Anspruch 5, **dadurch gekennzeichnet, dass** an einem der Ränder eines Abschnitts (253) der Krümmungswinkel (σ) der Rillen (251) kleiner ist als der Krümmungswinkel (σ') der Rillen (251) am anderen Rand des besagten Abschnitts.

8. Implantat nach wenigstens einem der Ansprüche 1 bis 7, **dadurch gekennzeichnet, dass** sich die Rillen (281) am Umfang von wenigstens einem von mehreren Abschnitten (282, 283 oder 284) befinden, die entlang einer gekrümmten Längsachse des Implantatkörpers (280) angeordnet sind und die entlang dieser ein keilförmiges Profil besitzen.

9. Implantat nach Anspruch 8, **dadurch gekennzeichnet, dass** die Rillen (281) am Umfang der besagten keilförmigen Abschnitte (282, 283 oder 284) eine unterschiedliche Länge aufweisen.

10. Implantat nach Anspruch 1 oder 5, **dadurch gekennzeichnet, dass** am Umfang von wenigstens einem der Abschnitte (263) erste Rillen (261) vorgesehen sind, die in einer ersten Richtung gewendelt verlaufen, und dass am Umfang des gleichen Abschnitts zweite Rillen (262) vorgesehen sind, die in einer zweiten Richtung gewendelt verlaufen und sich mit den ersten Rillen (261) kreuzen, und dass die ersten und die zweiten Rillen radial zur Längsachse des Implantatkörpers (11) mit dieser einen Winkel bilden und über ihre Länge eine unterschiedliche Breite und Tiefe aufweisen.

11. Implantat nach wenigstens einem der Ansprüche 1 bis 10, **dadurch gekennzeichnet, dass** zwischen den Abschnitten (272-275) in Umfangsrichtung verlaufende Bänder angeordnet sind, in deren Umfang die flachen Enden der Rillen (271) auslaufen.

12. Implantat nach einem der Ansprüche 1 bis 11, **dadurch gekennzeichnet, dass** die Rillen (251) in ihren Bereichen (255) größter Tiefe dicht benachbart sind und ein konkaves Profil aufweisen, das an seinen Rändern in Stege (256, 257) oder noppenförmige Erhöhungen am Umfang des Implantatkörpers übergeht.

13. Implantat nach Anspruch 1 oder 5, **dadurch gekennzeichnet, dass** der Implantatkörper (191) eine Kegelform aufweist und dass in Abschnitten (193) entlang des Kegels Rillen (190, 200) vorgesehen sind, die die Form von langgestreckten gekrümmten Segmenten (251) aufweisen.

14. Implantat nach Anspruch 1 oder 5 mit Abschnitten unterschiedlichen Durchmessers, die zur Bildung einer abgestuften Kegelform zum Implantatfuß hin abnehmen, **dadurch gekennzeichnet, dass** in den Abschnitten (272-275) entlang des abgestuften Kegels Rillen (271) vorgesehen sind, die die Form von langgestreckten gekrümmten Segmenten (251) aufweisen.

15. Implantat nach wenigstens einem der Ansprüche 1 bis 14, **dadurch gekennzeichnet, dass** am Umfang des Implantatkörpers (270) eine Kombination unterschiedlicher Rillenformen angeordnet ist.

16. Implantat nach Anspruch 15, **dadurch gekennzeichnet, dass** der Implantatkörper (270) Gruppen von Abschnitten (222, 223 und 224, 225, 272, 273 und 274, 275) aufweist und dass die Rillen (220) der einen Gruppe in ihrer Form von den Rillen (227, 271) der anderen Gruppe abweicht.

17. Implantat nach Anspruch 16, **dadurch gekennzeichnet, dass** die Rillen (220) der einen Gruppe (222, 223, 272, 273) kreuzweise gewendelt und die Rillen (227, 271) der anderen Gruppe in Richtung der Implantatlängsachse oder einfach gewendelt verlaufen.

18. Implantat nach Anspruch 1 oder 5, **dadurch gekennzeichnet, dass** sich die Rillen des obersten Abschnitts (272) in den Kopfbereich erstrecken bis zu einem Randbereich (276), der annähernd parallel zur kopfseitigen Begrenzung des Implantats verläuft.

19. Implantat nach wenigstens einem der Ansprüche 1 bis 18, **dadurch gekennzeichnet, dass** die Rillen (251, 261, 262) vom Umfang gemessen eine maximale Tiefe im Bereich von 150 Mikrometern aufweisen.

20. Implantat nach wenigstens einem der Ansprüche 1 bis 19, **dadurch gekennzeichnet, dass** die Rillen (251, 261, 262) eine maximale Breite im Bereich von 300 Mikrometern aufweisen.

21. Implantat nach wenigstens einem der Ansprüche 1 bis 20, **dadurch gekennzeichnet, dass** die Oberfläche der Rillen eine durch Sputtern hergestellte Metallschicht aufweist.

22. Implantat nach Anspruch 21, **dadurch gekennzeichnet, dass** die Metallschicht eine durch Sputtern hergestellte Titanschicht ist.

23. Implantat nach Anspruch 21, **dadurch gekennzeichnet, dass** sich die Metallschicht über die Zwischenräume (234, 256, 257) zwischen den Rillen erstreckt.

24. Implantat nach Anspruch 21 oder 22, **dadurch gekennzeichnet, dass** sich die Metallschicht über den Kopfbereich (12, 276) erstreckt.

25. Verfahren zum Herstellen eines Knochenimplantats, insbesondere eines Zahnimplantats, mit einem Implantatkörper (11), der an seinem Umfang eine Vielzahl in Richtung der Längsachse des Implantatkörpers oder in einem spitzen Winkel zu dieser verlaufende Rillen (20) aufweist, deren Größenordnung der Größenordnung der Osteone des Knochengewebes entspricht, die sich an die rillenförmigen Vertiefungen anlagern können, und dessen Oberfläche in Richtung seiner Längsachse in eine Anzahl Abschnitte (16) unterteilt ist, **dadurch gekennzeichnet, dass** ein Rohling des Implantatkörper (11, 270) hergestellt wird, in dem durch eine Materialabtragungsoperation in wenigstens einem der Abschnitte (252, 263, 272-275, 282-284) Rillen (255, 261, 262, 271, 281) eingebracht werden, die eine sich kontinuierlich verändernde Breite und Tiefe aufweisen, und dass die Rillen im abgewickelten Umfang einen annähernd rautenförmigen Grundriß oder die Form gekrümmter Segmente aufweisen und an beiden Rändern des besagten Abschnitts schmal und flach sind und dazwischen ihre maximale Breite und Tiefe aufweisen.

26. Verfahren nach Anspruch 25, **dadurch gekennzeichnet, dass** die Rillen durch Strählen hergestellt werden bei synchronem Vorschub von Implantatkörpers und Strählwerkzeug.

27. Verfahren nach Anspruch 25 und 26, **dadurch gekennzeichnet, dass** die Rillen (251) ihre maximale Breite und Tiefe in einem Bereich (255) aufweisen, der annähernd in der Mitte ihrer Längsausdehnung liegt.

28. Verfahren nach Anspruch 25 und 26, **dadurch gekennzeichnet, dass** die Rillen (251) ihre maximale Breite und Tiefe in einem Bereich aufweisen, der in Bezug auf einen der beiden Ränder in einem Drittel ihrer Längsausdehnung liegt.

29. Verfahren nach Anspruch 25, **dadurch gekennzeichnet, dass** der synchronem Vorschub so gewählt wird, dass die Rillen (251) in wenigstens einem der Abschnitte (252) in Umfangsrichtung eine Krümmung aufweisen.

30. Verfahren nach Anspruch 29, **dadurch gekennzeichnet, dass** an einem der Ränder des besagten Abschnitts (252) der Krümmungswinkel (σ) der Rillen (251) kleiner ist als der Krümmungswinkel (σ') der Rillen am anderen Rand des besagten Abschnitts (252).

31. Verfahren nach Anspruch 25, **dadurch gekennzeichnet, dass** der synchronem Vorschub so gewählt wird, dass die Rillen (130) über einen Teil eines der Abschnitte (16) radial zur Längsachse (41) des Implantatkörpers (11) einen ersten Winkel α bilden und über einen weiteren Teil des gleichen Abschnitts (16) radial zur Längsachse (41) des Implantatkörpers (11) einen zweiten Winkel ß bilden.

32. Verfahren nach Anspruch 31, **dadurch gekennzeichnet, dass** die Rillen (140) am Umfang des Implantatkörpers gewendelt verlaufen.

33. Verfahren nach wenigstens einem der Ansprüche 25 bis 32, **dadurch gekennzeichnet, dass** der synchrone vorschub von Abschnitt zu Abschnitt verändert wird.

34. Verfahren nach wenigstens einem der Ansprüche 25 bis 33, **gekennzeichnet durch** den Schritt, dass zwischen den Abschnitten (203) in Umfangsrichtung verlaufende Bänder (192) erzeugt werden, in deren Umfang die flachen Enden der Rillen (200) auslaufen.

35. Verfahren nach wenigstens einem der Ansprüche 25 bis 33, **dadurch gekennzeichnet, dass** der synchronem Vorschub so gewählt wird, dass die Rillen benachbarter Abschnitte (212-215) aneinander anschließen.

36. Verfahren nach wenigstens einem der Ansprüche 25 bis 34, **dadurch gekennzeichnet, dass** die Rillen (271) des kopfseitigen Abschnitts (272) in den Kopfbereich erweitert werden bis zu einem Randbereich (276), der annähernd parallel zur kopfseitigen Begrenzung des Implantats verläuft.

37. Verfahren nach wenigstens einem der Ansprüche 25 bis 36, **gekennzeichnet durch** den zusätzlichen Schritt, dass auf die Oberfläche der Rillen eine Metallschicht **durch** Sputtern aufgebracht wird.

38. Verfahren nach Anspruch 37, **dadurch gekennzeichnet, dass** die Metallschicht eine durch Sputtern hergestellte Titanschicht ist.

39. Verfahren nach Anspruch 37 oder 38, **dadurch gekennzeichnet, dass** sich die Metallschicht über die Zwischenräume zwischen den Rillen erstreckt.

40. Verfahren nach Anspruch 37 oder 38, **dadurch gekennzeichnet, dass** sich die Metallschicht über die radial verlaufenden Bänder (163, 192, 226) zwischen den Abschnitten erstreckt.

41. Verfahren nach Anspruch 37 oder 38, **dadurch gekennzeichnet, dass** sich die Metallschicht über den Kopfbereich (276) des Implantatkörpers erstreckt.

## Claims

1. Bone implant, in particular a dental implant, with an implant body (11) which comprises at its periphery a plurality of grooves (20) having a direction along the implant body longitudinal axis or at an acute angle thereto and having a dimension which corresponds to the dimension of the bone tissue osteons which may attach to the groove-shaped recesses; and wherein the surface of the implant body is divided along its lengthwise axis into a plurality of sections (16), **characterized in that** in at least one of the sections the grooves (130, 140, 200) comprises in the development of the perimeter of the section an approximate diamond-shaped outline so that the grooves (251) are small and flat at both rims of the section and have in between their maximum depth and width.

2. Implant according to claim 1, **characterized in that** between the sections (203) in the direction of their periphery radial bands (192) are arranged in the perimeter of which the flat ends of the grooves (200) run out.

3. Implant according to claim 1, **characterized in that** the grooves of adjacent sections (212-215) join to each other.

4. Implant according to claim 1, **characterized in that** the basis of the grooves (130) form a first angle α radial to the longitudinal axis (41) of implant body (11) across a portion of a section (16) and a second angle ß radial the longitudinal axis (41) of the implant body (11) across another portion of the same section (16) which second angle is oriented in contrary direction to the first angle.

5. Bone implant, in particular a dental implant, with an implant body (11) which comprises at its periphery a plurality of grooves (20) having a direction along the implant body longitudinal axis or at an acute angle thereto and having a dimension which corresponds to the dimension of the bone tissue osteons which may attach to the groove-shaped recesses; and wherein the surface of the implant body is divided along its lengthwise axis into a plurality of sections (16), **characterized in that** in at least one of the sections (252, 242) the grooves (251, 261, 262) comprise a varying width and depths; and that the grooves exhibits at the development of the perimeter of the section a shape in form of elongated curved segments which are small and shallow at both edges of the section and have in between their maximum depth and width.

6. Implant according to claim 1 or 5, **characterized in that** the grooves (251) have their maximum width and depth in an area (255) which is in close proximity to the middle of their longitudinal extension.

7. Implant according to claim 5, **characterized in that** at one edge of a section (253) the angle of the curvature (o) of the grooves (251) is smaller than the angle of curvature (o') of the grooves (251) at the other edge of the section.

8. Implant according to at least one of the claims 1 to 7, **characterized in that** the grooves (281) are disposed at the perimeter of at least one of a number of sections (282, 283 or 284) which are arranged along a curved longitudinal axis of the implant body (280) and which have along those a wedge-shaped profile.

9. Implant according to claim 8, **characterized in that** the grooves (281) at the perimeter of said wedge-shaped sections (282, 283 or 284) have differing lengths.

10. Implant according to claim 1 or 5, **characterized in that** it comprises first grooves (261) at the perimeter of at least one of the sections and oriented in a first winding direction, and further comprises second grooves (262) which are arranged at the perimeter of the same section and oriented to a second winding direction and which intersect with the first grooves (261); and wherein the first and the second grooves form radial to the longitudinal axis of implant body (11) with those an angle and have varying widths and depths across their lengths.

11. Implant according to at least one of the claims 1 to 10, **characterized in that** between the sections (272-275) in the direction of their periphery radial bands are arranged where in the perimeter of which the flat ends of the grooves (271) run out.

12. The implant according one of the claims 1 to 11, **characterized in that** the grooves (251) are arranged densely adjacent at their areas (255) of greatest depth and comprises a concave profile the edges of which pass over to spans (256, 257) or knob-shaped protrusions at the perimeter of implant body (11).

13. Implant according to claim 1 or 5, **characterized in that** the implant body (191) comprises the form of a cone and that in sections (193) along the cone grooves (190, 200) are arranged along the cone which groooves have the form of elongated curved segments (251).

14. Implant according to claim 1 or 5 with sections of differing diameters decreasing toward the bottom end of the implant body to form a stepped conical shape, **characterized in that** in the sections (272-275) along the stepped cone grooves (271) are arranged which have the form of elongated curved segments (251).

15. Implant according to at least one of the claims 1 to 14, **characterized in that** a combination of different forms of said grooves is arranged at the perimeter of the implant body (270).

16. Implant according to claim 15, **characterized in that** the implant body (270) comprises groups of sections (222, 223 and 224, 225, 272, 273 and 274, 275) and that the grooves (220) of one group differ in their form from the grooves (227, 271) of another group (224, 225, 273, 274).

17. Implant according to claim 16, **characterized in that** the grooves (220) of one group (222, 223, 272, 273) are spiral crosswise are arranged and the grooves (227, 271) of another group run along the implant longitudinal axis or in a single spiral direction.

18. Implant according to claims 1 or 5, **characterized in that** the grooves of the topmost section (272) extend to the top end of the implant body to an edge region (276) which runs approximately parallel to the top end of the implant body.

19. Implant according to at least one of the claims 1 to 18, **characterized in that** the grooves (251, 261, 262) have a maximum depth, measured at the perimeter, in the range of 150 micrometers.

20. Implant according to at least one of the claims 1 to 19, **characterized in that** the grooves (251, 261, 262) have a maximum width in the range of 300 micrometers.

21. Implant according to at least one of the claims 1 to 20, **characterized in that** the surface of the grooves comprises a metal layer produced by sputtering.

22. Implant according to claim 21, **characterized in that** the metal layer comprises a titanium coating produced by metal sputtering.

23. Implant according to claim 21, **characterized in that** the metal layer extends across the spaces (234, 256, 257) between the grooves.

24. Implant according to claim 21 or 22, **characterized in that** the metal layer extends over the head area (12, 276).

25. Method of manufacturing a bone implant, in particular a dental implant, with an implant body (11) which comprises at its periphery a plurality of grooves (20) having a direction along the implant body longitudinal axis or at an acute angle thereto and having a dimension which corresponds to the dimension of the bone tissue osteons which may attach to the groove-shaped recesses; and wherein the surface of the implant body is divided along its lengthwise axis into a plurality of sections (16), **characterized in that**:
forming a blank of the implant body (11, 270) in which grooves (225, 261, 262, 271, 281) at the perimeter of at least one of said sections (252, 263, 272-275, 282-294) are produced by a material removing process; the grooves comprise a continuously varying width and depths; and
that the grooves comprises in the development of the perimeter an approximate diamond-shaped outline or the form of elongated curved segments and are small and shallow at both edges of the section and have in between their maximum depth and width.

26. Method according to claim 25, **characterized in that** the material removing process is a metal cutting process using a synchronous feed of the implant body and the cutting tool.

27. Method according to claim 25 and 26, **characterized in that** the grooves (251) comprise their maximum width and depth in an area (255) which is located close to the center of their lengthwise extension.

28. Method according to claim 25 and 26, **characterized in that** the grooves (251) comprise their maximum width and depth in an area which is located at one-third of their lengthwise extension relative one of the two edges.

29. Method according to claim 25, **characterized in that** the synchronous feed is selected in such way that the grooves (251) comprise a peripheral curvature in at least one of the sections (252).

30. Method according to claim 29, **characterized in that** the angle of curvature (σ) of grooves (251) is smaller at one of the edges of said section (252) than the angle of curvature (σ') of grooves at the other edge of said section (252).

31. Method according to claim 25, **characterized in that** the synchronous feed is selected in such way that the grooves (130) form over a part of one of the sections (16) a first angle α radial to the longitudinal axis (41) of the implant body (11) and a second angle ß radial the longitudinal axis (41) of the implant body (11) over another part of the same section (16).

32. Method according to claim 31, **characterized in that** the grooves (140) extend in spiral fashion at the perimeter of the section.

33. Method according to at least one of the claims 25 to 32, **characterized in that** the synchronous feed changes from section to section.

34. Method according to at least one of the claims 25 to 33, **characterized by** the step in that between the sections (203) in the direction of their periphery radial bands (192) are generated in the perimeter of which the flat ends of the grooves (200) run out.

35. Method according to at least one of the claims 25 to 33, **characterized in that** the synchronous feed is selected in such way that the grooves of adjacent sections (212-215) join to each other.

36. Method according to at least one of the claims 25 to 34, **characterized in that** the that the grooves (271) of the topmost section (272) extend to the top end of the implant body to an edge region (276) which runs approximately parallel to the top end of the implant body.

37. Method according to at least one of the claims 25 to 36, **characterized by** the further step of applying a metal layer on the surface of the grooves by sputtering.

38. Method according to claim 37, **characterized in that** the metal layer is a titanium coating produced by sputtering.

39. Method according to claim 37 or 38, **characterized in that** the metal layer extends across the spaces between the grooves.

40. Method according to claim 37 or 38, **characterized in that** the metal layer extends across the radial bands (163, 192, 226) between the sections.

41. Method according to claim 37 or 38, **characterized in that** the metal layer extends over the head area (276) of the implant body.

## Revendications

1. Implant osseux, notamment implant dentaire avec un corps d'implant (11) qui présente sur sa circonférence une multitude de rainures (20) s'étendant dans le sens de l'axe longitudinal du corps d'implant ou selon un angle aigu par rapport à l'axe longitudinal, dont la dimension correspond à la dimension des ostéones du tissu osseux, lesquelles peuvent se déposer contre les creux rainurés et dont la surface est subdivisée dans le sens de son axe longitudinal en une série de tronçons (16) **caractérisé en ce que** les rainures (130, 140, 200) présentent sur au moins un des tronçons sur la circonférence développée un tracé quasiment rhombique, de sorte que les rainures (251) sur les deux bords d'un tronçon soient étroites et planes et présentent entre ces bords leur largeur et profondeur maximales.

2. Implant selon la revendication 1 **caractérisé en ce qu'**il est disposé entre les tronçons (203) des bandes (192) s'étendant dans le sens de la circonférence, dans la circonférence desquelles se fondent les extrémités planes des rainures (200).

3. Implant selon la revendication 1 **caractérisé en ce que** les rainures de tronçons contigus (212-215) se rejoignent.

4. Implant selon la revendication 1 **caractérisé en ce que** la base des rainures (130) est inclinée radialement par rapport à l'axe longitudinal (41) du corps d'implant (11) sur une partie d'un tronçon (16) selon un premier angle ∝ et est inclinée radialement par rapport à l'axe longitudinal (41) sur une autre partie du même tronçon (16) selon un second angle β, lequel est opposé au premier angle.

5. Implant osseux, notamment implant dentaire avec un corps d'implant (11) qui présente sur sa circonférence une multitude de rainures (20) s'étendant dans le sens de l'axe longitudinal du corps d'implant ou selon un angle aigu par rapport à l'axe longitudinal, dont la dimension correspond à la dimension des ostéones du tissu osseux, lesquelles peuvent se déposer contre les creux rainurés et dont la surface est subdivisée dans le sens de son axe longitudinal en une série de tronçons (16) **caractérisé en ce que** les rainures (251, 261, 262) présentent sur au moins un des tronçons (252, 242) une largeur et une profondeur continuellement changeantes et **en ce que** le tracé des rainures sur la circonférence développée présente la forme de segments courbés, qui sont étroits et planes sur les deux bords dudit tronçon et présentent entre ces bords leur largeur et profondeur maximales.

6. Implant selon la revendication 1 ou 5, **caractérisé en ce que** les rainures (251) présentent leur largeur et profondeur maximales dans une zone (255), située approximativement au centre de leur étendue longitudinale.

7. Implant selon la revendication 5 **caractérisé en ce que**, sur l'un des bords du tronçon (253), l'angle de courbure (σ) des rainures (251) est plus petit que l'angle de courbure (σ') des rainures (251) sur l'autre bord dudit tronçon.

8. Implant selon au moins une des revendications 1 à 7 **caractérisé en ce que** les rainures (281) se trouvent sur la circonférence d'au moins un parmi plusieurs tronçons (282, 283 ou 284) qui sont disposés le long d'un axe longitudinal courbé du corps d'implant (280) et qui possèdent le long de celui-ci un profil de forme conique.

9. Implant selon la revendication 8 **caractérisé en ce que** les rainures (281) présentent sur la circonférence desdits tronçons (282, 283 ou 284) de forme conique une longueur différente.

10. Implant selon la revendication 1 ou 5 **caractérisé en ce qu'**il est prévu sur la circonférence d'au moins un des tronçons (263) des premières rainures (261) qui s'étendent en spirale dans une première direction et **en ce qu'**il est prévu sur la circonférence du même tronçon des secondes rainures (262) qui s'étendent en spirale dans une seconde direction et se croisent avec les premières rainures (261) et **en ce que** les premières et les secondes rainures forment radialement par rapport l'axe longitudinal du corps d'implant (11) un angle avec celles-ci et présentent sur leurs longueurs une largeur et une profondeur différentes.

11. Implant selon au moins une des revendications 1 à 10 **caractérisé en ce qu'**il est disposé entre les tronçons (272-275) des bandes s'étendant dans le sens de la circonférence, dans la circonférence desquelles se fondent les extrémités planes des rainures (271).

12. Implant selon l'une des revendications 1 à 11 **caractérisé en ce que** les rainures (251) sont étroitement contiguës dans leurs zones (255) de profondeur maximale et présentent un profil concave qui se prolonge sur ses bords en des nervures (256, 257) ou des élévations en forme de nopes sur la circonférence du corps d'implant.

13. Implant selon la revendication 1 ou 5, **caractérisé en ce que** le corps d'implant (191) présente une forme conique et **en ce que** des rainures (190, 200) sont prévues dans les tronçons (193) le long du cône, présentant la forme de segments (251) courbés s'étirant en longueur.

14. Implant selon la revendication 1 ou 5 avec des tronçons de différents diamètres qui se réduisent vers la base de l'implant afin de former une forme conique étagée **caractérisé en ce que** des rainures (271) sont prévues dans les tronçons (272-275), le long du cône étagé, présentant la forme de segments (251) courbés s'étirant en longueur.

15. Implant selon au moins une des revendications 1 à 14, **caractérisé en ce qu'**une combinaison de rainurages différents est disposés sur la circonférence du corps d'implant (270) .

16. Implant selon la revendication 15 **caractérisé en ce que** le corps d'implant (270) présente des groupes de tronçons (222, 223 et 224, 225, 272, 273 et 274, 275) et **en ce que** les rainures (220) de l'un des groupes diverge par sa forme des rainures (227, 271) de l'autre groupe.

17. Implant selon la revendication 16 **caractérisé en ce que** les rainures (220) de l'un des groupes (222, 223, 272, 273) s'étendent en spirale croisée et que les rainures (227, 271) de l'autre groupe s'étendent dans le sens de l'axe longitudinal de l'implant ou en spirale simple.

18. Implant selon la revendication 1 ou 5 **caractérisé en ce que** les rainures du tronçon supérieur (272) s'étendent dans la zone de tête jusqu'à une zone en bordure (276) qui s'étend quasiment parallèlement à la limite de l'implant du côté tête.

19. Implant selon au moins une des revendications 1 à 18 **caractérisé en ce que** les rainures (251, 261, 262), mesurées à partir de la circonférence, présentent une profondeur maximale de l'ordre de 150 micromètres.

20. Implant selon au moins une des revendications 1 à 19 **caractérisé en ce que** les rainures (251, 261, 262) présentent une largeur maximale de l'ordre de 300 micromètres.

21. Implant selon au moins une des revendications 1 à 20 **caractérisé en ce que** la surface des rainures présente une couche métallique fabriquée par pulvérisation cathodique.

22. Implant selon la revendication 21 **caractérisé en ce que** la couche métallique est une couche de titane fabriquée par pulvérisation cathodique.

23. Implant selon la revendication 21 **caractérisé en ce que** la couche métallique s'étend sur les espaces intermédiaires (234, 256, 257) entre les rainures.

24. Implant selon la revendication 21 ou 22 **caractérisé en ce que** la couche métallique s'étend sur la zone de tête (12, 276).

25. Procédé de fabrication d'un implant osseux, notamment d'un implant dentaire, avec un corps d'implant (11) qui présente sur sa circonférence une multitude de rainures (20) s'étendant dans le sens de l'axe longitudinal du corps d'implant ou selon un angle aigu par rapport à l'axe longitudinal, dont la dimension correspond à la dimension des ostéones du tissu osseux, lesquelles peuvent se déposer contre les creux rainurés et dont la surface est subdivisée dans le sens de son axe longitudinal en une série de tronçons (16) **caractérisé en ce qu'**une ébauche du corps d'implant (11, 270) est fabriquée sur laquelle sont positionnées, lors d'une opération d'enlèvement de matière, des rainures (255, 261, 262, 271, 281) sur au moins un des tronçons (252, 263, 272-275, 282-284) qui présentent une largeur et une profondeur continuellement changeantes et **en ce que** les rainures sur la circonférence développée présentent un tracé quasiment rhombique ou la forme de segments courbés et sont étroits et planes sur les deux bords dudit tronçon et présentent entre ces bords leur largeur et profondeur maximales.

26. Procédé selon la revendication 25 **caractérisé en ce que** les rainures sont fabriqués par rayonnement avec une avancée synchrone du corps d'implant et de l'outil de rayonnement.

27. Procédé selon la revendication 25 et 26 **caractérisé en ce que** les rainures (251) présentent leur largeur et profondeur maximales dans une zone (255) située approximativement au centre de leur étendue longitudinale.

28. Procédé selon la revendication 25 et 26 **caractérisé en ce que** les rainures (251) présentent leur largeur et profondeur maximales dans une zone située, par rapport à l'un des deux bords, à un tiers de leur étendue longitudinale.

29. Procédé selon la revendication 25 **caractérisé en ce que** l'avancée synchrone est sélectionnée de telle sorte que les rainures (251) présentent sur au moins un des tronçons (252) une courbure dans le sens de la circonférence.

30. Procédé selon la revendication 29 **caractérisé en ce que** sur l'un des bords dudit tronçon (252), l'angle de courbure (σ) des rainures (251) est plus petit que l'angle de courbure (σ') des rainures (251) sur l'autre bord dudit tronçon (252).

31. Procédé selon la revendication 25 **caractérisé en ce que** l'avancée synchrone est sélectionnée de telle sorte que les rainures (130) forment sur une partie d'un des tronçons (16) un premier angle ∝ radial par rapport à l'axe longitudinal (41) et forment sur une autre partie du même tronçon (16) un second angle β radial par rapport à l'axe longitudinal (41) du corps d'implant (11).

32. Procédé selon la revendication 31 **caractérisé en ce que** les rainures (140) s'étendent en spirale sur la circonférence du corps d'implant.

33. Procédé selon au moins une des revendications 25 à 32 **caractérisé en ce que** l'avancée synchrone est modifiée de tronçon en tronçon.

34. Procédé selon au moins une des revendications 25 à 33 **caractérisé par** l'étape qui consiste à produire des bandes (192) s'étendant entre les tronçons (203) dans le sens de la circonférence, dans la circonférence desquelles se fondent les extrémités planes des rainures (200).

35. Procédé selon au moins une des revendications 25 à 33 **caractérisé en ce que** l'avancée synchrone est sélectionnée de telle sorte que les rainures de tronçons contigus (212-215) se rejoignent.

36. Procédé selon au moins une des revendications 25 à 34 **caractérisé en ce que** les rainures (271) du tronçon (272) du côté tête sont élargies dans la zone de tête jusqu'à une zone périphérique (276) qui s'étend quasiment parallèlement à la limite côté tête de l'implant.

37. Procédé selon au moins une des revendications 25 à 36 **caractérisé par** l'étape supplémentaire qui consiste à appliquer sur la surface des rainures une couche métallique par pulvérisation cathodique.

38. Procédé selon la revendication 37 **caractérisé en ce que** la couche métallique est une couche de titane fabriquée par pulvérisation cathodique.

39. Procédé selon la revendication 37 ou 38 **caractérisé en ce que** la couche métallique s'étend sur les espaces intermédiaires entre les rainures.

40. Procédé selon la revendication 37 ou 38 **caractérisé en ce que** la couche métallique s'étend sur les bandes (163, 192, 226) s'étendant radialement, entre les tronçons.

41. Procédé selon la revendication 37 ou 38 **caractérisé en ce que** la couche métallique s'étend sur la zone de tête (276) du corps d'implant.
